# EUROPEAN PATENT SPECIFICATION

(11) **EP 0 467 956 B1**
(45) Date of publication and mention of the grant of the patent: **09.02.1994**
(21) Application number: 90906543.5
(22) Date of filing: 12.04.1990
(51) Int. Cl.: A61B 5/11

(54) **APPARATUS FOR MONITORING THE MOTION COMPONENTS OF THE SPINE**
GERÄT ZUR ÜBERWACHUNG DER BEWEGUNGSKOMPONENTEN DER WIRBELSÄULE
APPAREIL DE CONTRôLE DES COMPOSANTS DE MOUVEMENT DE LA COLONNE VERTEBRALE

(30) Priority: 12.04.1989 US 336896
(43) Date of publication of application: 29.01.1992
(73) Proprietor: MARRAS, William S., Powell, OH 43065 (US)
(72) Inventor: MARRAS, William, S., Powell, OH 43065 (US); DAVIS, Shelby, W., Amlin, OH 43002 (US); MILLER, Robert, J., Westerville, OH 43081 (US); MIRKA, Gary, A., Brecksville, OH 44141 (US)
(74) Representative: Lieck, Hans-Peter, Dipl.-Ing.
(86) International application number: US9001992
(87) International publication number: WO9011720

(56) References cited:
- WO-A-87/00026
- FR-A- 2 590 151
- SE-A- 8 605 103
- US-A-36 085 41

## Description

### Background of the Invention

The present invention relates to an apparatus adapted to be mounted on the back of a patient and for monitoring the motion components of the spine during movement of the patient.

In diagnosing physical injuries or deterioration of the human spine, it is common to utilize a small goniometer which is attached on the back of the patient, and which indicates the static posture of the patient. Such instruments are adequate for documenting the bend or curvature of the spine, but they are incapable of documenting the motion or velocity or acceleration components, which are also useful in diagnosis.

More sophisticated diagnostic devices are also known which are capable of measuring the velocity components of the spine during movement of the patient. These velocity measuring devices are designed to permit the patient to physically enter the device, and thus they are very large and quite expensive. Also, in view of their size, these devices have a substantial moment of inertia which tends to mask the motion patterns of the patient.

PCT Publication No. WO 89/11247 discloses a device for detecting spinal movements and which includes a column unit composed of a number of separate vertebrae having three draw bars and a torsion rod extending therethrough. The column unit is attached to the patient's back by means of an elastic band having pins which fit in recesses in the vertebrae. The band is provided with an adhesive on the reverse side, so that it sticks to the patient's back, and the device thus responds to the movement of individual vertebrae of the patient.

It is accordingly an object of the present invention to provide a relatively simple and inexpensive apparatus for monitoring the motion components of the human spine, and which is useful in diagnosing a physical injury or deterioration of the spine.

It is also an object of the present invention to provide an apparatus of the described type and which is adapted to be mounted on the back of the patient so as not to interfere with normal body movements, and which can be worn "on the job" so as to permit an analysis of the motion components which are required to be performed by a worker.

It is a further object of the present invention to provide an apparatus of the described type which monitors the collective movements of the lumbar vertebrae of the patient.

### Summary of the Invention

These and other objects and advantages of the present invention are achieved in the embodiment illustrated herein by the provision of an apparatus which comprises an elongate exoskeleton comprising a plurality of generally flat elements disposed in a longitudinally spaced apart arrangement. A spacer is disposed between each adjacent pair of elements for maintaining the separation of the elements, and the spacers are longitudinally aligned and define a central longitudinal axis. A plurality of openings extend longitudinally through each of said elements, with each of said openings of each element being spaced from the central axis and being longitudinally aligned with a corresponding opening of the remaining elements.

The apparatus further comprises support means for supporting the exoskeleton on the back of a patient and so that the exoskeleton is generally aligned along the spine. The support means comprises a top plate, strap means for releasably securing the top plate on the upper back of the patient, a base plate, belt means for releasably securing said base plate on the lower back of the patient. The support means further comprises a support plate which slideably mounts the top plate so as to permit movement in the longitudinal direction, and spring biasing means for resiliently urging the top plate in a direction away from the base plate. Extensible means extends longitudinally through each of the aligned openings of the elements, with each of said extensible means being resiliently extensible in length, and with the opposite ends of each extensible means being respectively attached to the top plate and the base plate. Thus, flexing movement of the exoskeleton caused by the movement of the patient causes each of the extensible means to selectively change in length. The apparatus also includes movement signaling means mounted to the support means for detecting longitudinal movement of each of the extensible means caused by changes in the length thereof, and for providing an output signal representative of such movement.

In the preferred embodiment as illustrated herein, there are three openings in each of the elements and which are disposed in a triangular arrangement. Also, each of the elements further includes a bore extending therethrough which is aligned with the central axis, and a cable means extends longitudinally through the bores of the elements. One end of the cable means is fixedly mounted to the top plate, and the other end is rotatably mounted to the base plate. Further, a rotation signaling means is mounted to the base plate for detecting rotation of the cable means in either direction and providing an output signal representative thereof.

The present invention thus permits three planes of movement to be simultaneously monitored, the sagittal (i.e. forward and back), transverse (i.e. twisting), and the lateral planes. In accordance with a further aspect of the invention, the monitored motion characteristics in each such plane include the angular position of the spine as a function of time, the angular velocity as a function of time, and the angular acceleration as a function of time.

### Brief Description of the Drawings

Some of the objects and advantages of the present invention having been stated, others will appear as the description proceeds, when taken in conjunction with the accompanying drawings, in which
Figure 1 is a perspective view illustrating the apparatus of the present invention mounted on the back of a patient during use;
Figure 2 is a fragmentary perspective view of the apparatus;

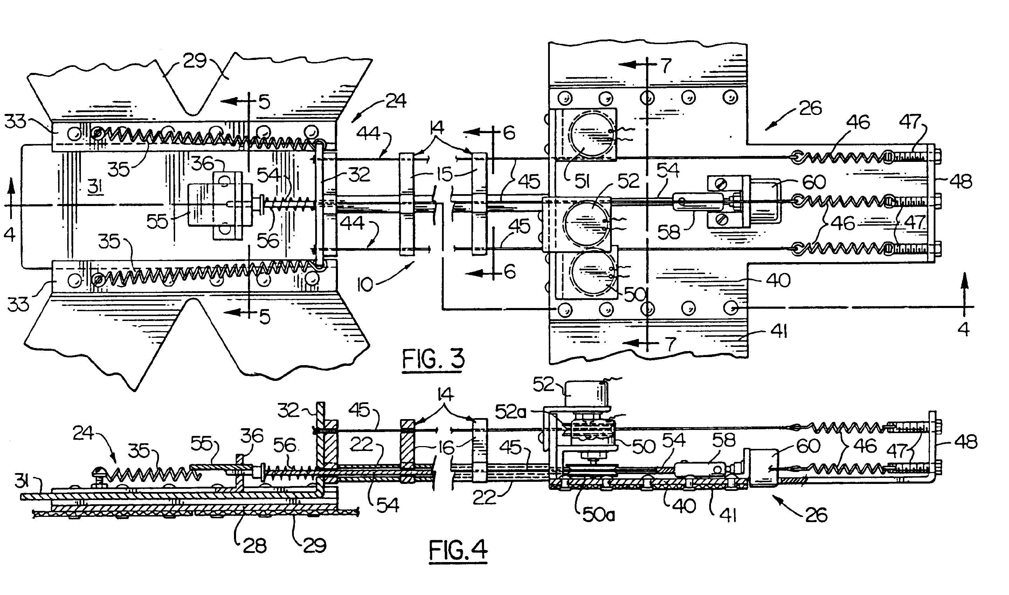

Figures 3 and 4 are top plan and side elevation views of the apparatus respectively;
Figures 5, 6, and 7 are sectional views taken respectively along the lines 5-5, 6-6, and 7-7 of Figure 3;
Figure 8 is a schematic flow diagram illustrating a preferred embodiment of the signal processing system of the present invention; and
Figure 9, 10, and 11 are diagrams illustrating a typical output of the angular position, velocity, and acceleration as a function of time, respectively.

### Detailed Description of the Preferred Embodiment

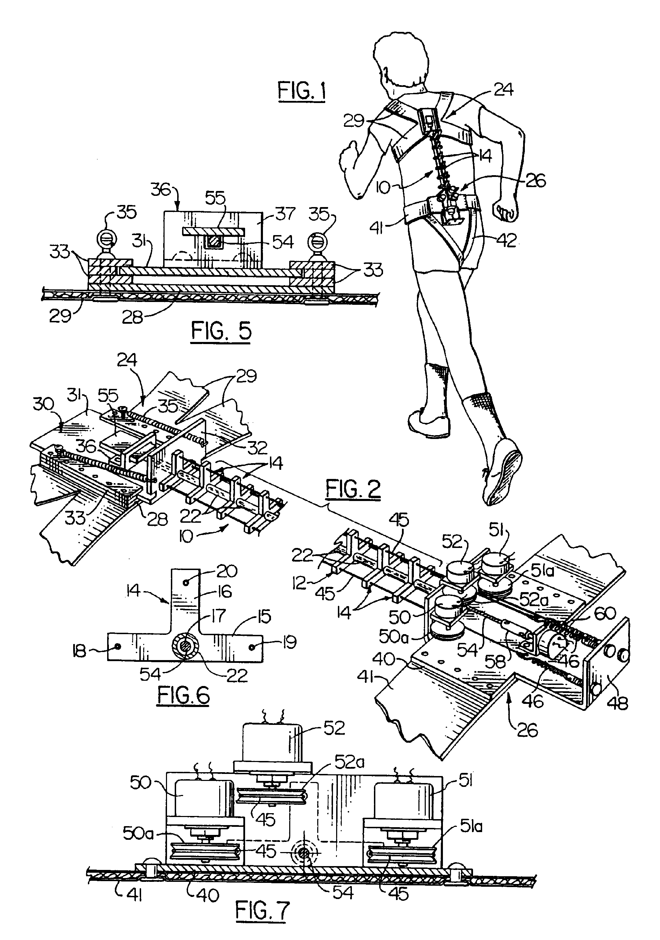

Referring more particularly to the drawings, Figure 1 illustrates an apparatus 10 which embodies the present invention and which is shown mounted on the back of a patient. In the illustrated embodiment, the apparatus is used to monitor the movement of the spine, and particularly the lumbar spine.

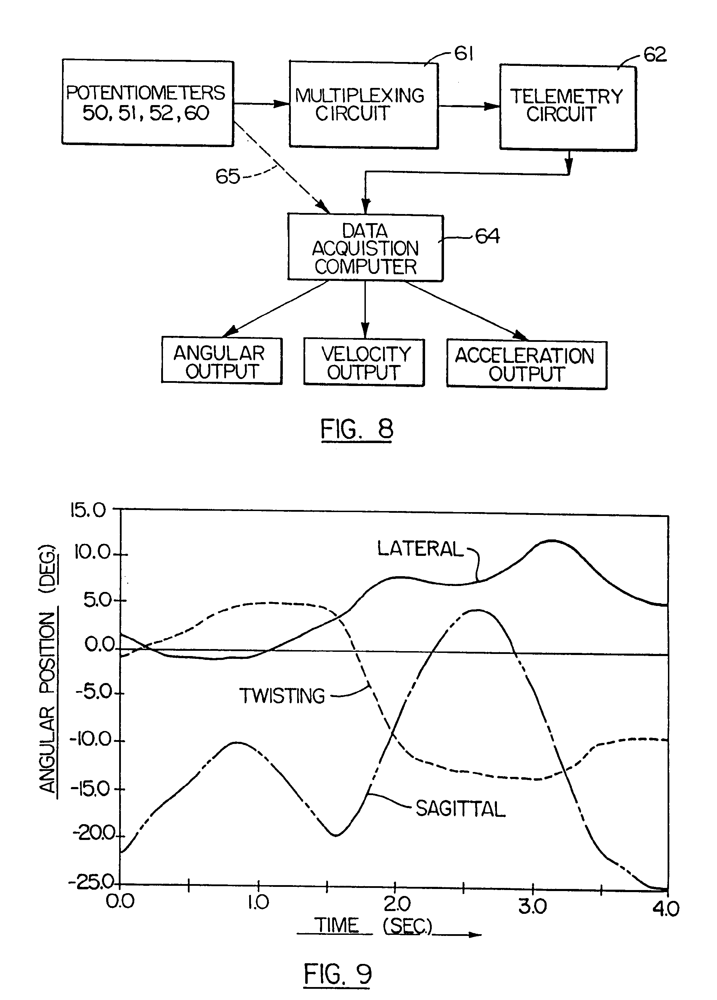

The apparatus 10 includes an elongate exoskeleton 12 comprising a plurality of generally flat, T-shaped elements 14 which are disposed in a longitudinally spaced apart arrangement. As best seen in Figure 6, each element 14 is composed of a horizontal base leg 15, and an upright leg 16. Also, each element includes a central bore 17 at the intersection of the two legs, with the bore 17 extending longitudinally therethrough. Further, first and second smaller openings 18, 19 are positioned at the respective ends of the horizontal base leg on opposite sides of the central bore 17, and such that the bore 17 and the first and second openings 18, 19 lie substantially in a common horizontal plane. A third opening 20 is provided adjacent the free end of the upright leg 16, and such that the third opening 20 is equally spaced from the first and second openings, and the first, second, and third openings are disposed in a triangular arrangement.

The elements 14 are each disposed in parallel transverse planes, and they are aligned along the longitudinal direction. The central bores 17 of the elements are also longitudinally aligned to define a central longitudinal axis. Similarly, the first, second, and third openings of each element are longitudinally aligned with the corresponding openings of the other elements.

A tubular spacer 22 is disposed between each adjacent pair of elements 14 for maintaining the separation thereof, and with the spacers 22 being longitudinally aligned with the central bores and thus with the central axis. The spacers 22 are not fixedly attached to the elements, but are supported in the described position by the structure set forth below, and so as to permit relative motion of the elements 14 in all axes.

The apparatus 10 also includes support means for supporting the exoskeleton 12 on the back of a patient and so that the exoskeleton is generally aligned along the spine as best seen in Figure 1. This support means includes an upper assembly 24, and a lower assembly 26. More particularly, the upper assembly 24 includes a support plate 28, which is fixedly attached to a harness 29 which is in the form of a figure eight and which loops about the shoulders of the patient. The harness 29 is preferably fabricated from a semi-rigid material, such as "Orthoplast" material sold by Johnson & Johnson, and which is open in the front, and with the front being closed by suitable straps having "Velcro" type interconnection means (not shown). This construction provides a stable mounting of the exoskeleton 12 to the upper body of the wearer.

The upper assembly 24 also includes a top plate 30 which is slideably connected to the support plate 28. In this regard, the top plate 30 has an L-shaped configuration so as to include a flat lower segment 31 and an upright segment 32. The support plate 28 includes opposing side members 33 which define opposing slots, and the lower segment 31 of the top plate fits within the slots as best seen in Figure 5, and so that the top plate is free to slide in the longitudinal direction with respect to the support plate. A pair of springs 35 extend between the side members 33 and the upright segment 32 of the top plate, and act to bias the top plate upwardly with respect to the support plate and the strap. Also, the lower segment 31 of the top plate mounts a bracket 36 having an upright leg 37 which contains a horizontal slot, for the purposes described below.

The lower assembly 26 includes a base plate 40 which is mounted to a belt 41, and such that the belt releasably secures the base plate on the lower back of the patient. In the illustrated embodiment, the belt 41 comprises a waist band which may also be composed of "Orthoplast" material, and which may be divided in front and include "Velcro" type interconnection means (not shown). Also, a pair of straps 42 are attached to the waist band and extend between the legs of the patient.

The apparatus 10 of the present invention also includes extensible means 44 extending longitudinally through each of the three aligned openings 18, 19, 20 of the elements 14, and with each of the extensible means 44 being resiliently extensible in length. More particularly, each of the extensible means includes a non-extensible solid wire 45, and an extensible spring 46 attached to one end of the wire. Each wire 45 has its opposite end fixed to the upright segment 32 of the top plate 30, and each wire 45 runs through all of the elements and to a point adjacent the lower portion of the base plate 40 where it joins one end of the spring 46. The opposite end of the spring 46 is connected to a threaded member 47, which in turn is joined to the upright flange 48 of the base plate 40.

Movement signaling means is mounted on the base plate for detecting longitudinal movement of each of the wire means caused by changes in length thereof, and for providing an output signal representative of such movement. In the illustrated embodiment, this signaling means comprises three electrical potentiometers 50, 51, 52 mounted to the base plate. Each potentiometer includes a pulley 50a, 51a, 52a respectively, and the associated wire 45 is wrapped about the pulley so that any longitudinal movement of the wire with respect to the base plate causes the associated pulley and potentiometer to rotate.

The apparatus 10 further includes a cable 54 extending longitudinally through the aligned bores 17 of the segments and coaxially through the spacers 22. The cable 54, which preferably comprises a piano wire, has one end attached to a plate 55 which is slideably received in the slot of the bracket 36 as best seen in Figure 5, and a spring 56 is interposed between the end of the cable and the upright segment 32 of the top plate. The slideable interconnection formed between the plate 55 and the bracket 36 accommodates the elongation of the spine which occurs when the patient bends forward, while maintaining cable 54 in a fixed rotational orientation at its upper end. The opposite end of the cable 54 is fixed to a connector 58, and the connector 58 in turn is fixed to the output shaft of a fourth potentiometer 60 mounted on the base plate 40. Thus any twisting of the exoskeleton about the central axis causes the potentiometer 60 to rotate. As will be understood by those skilled in the art, other movement sensors may be employed as an alternative to the illustrated potentiometers, such as optical encoders.

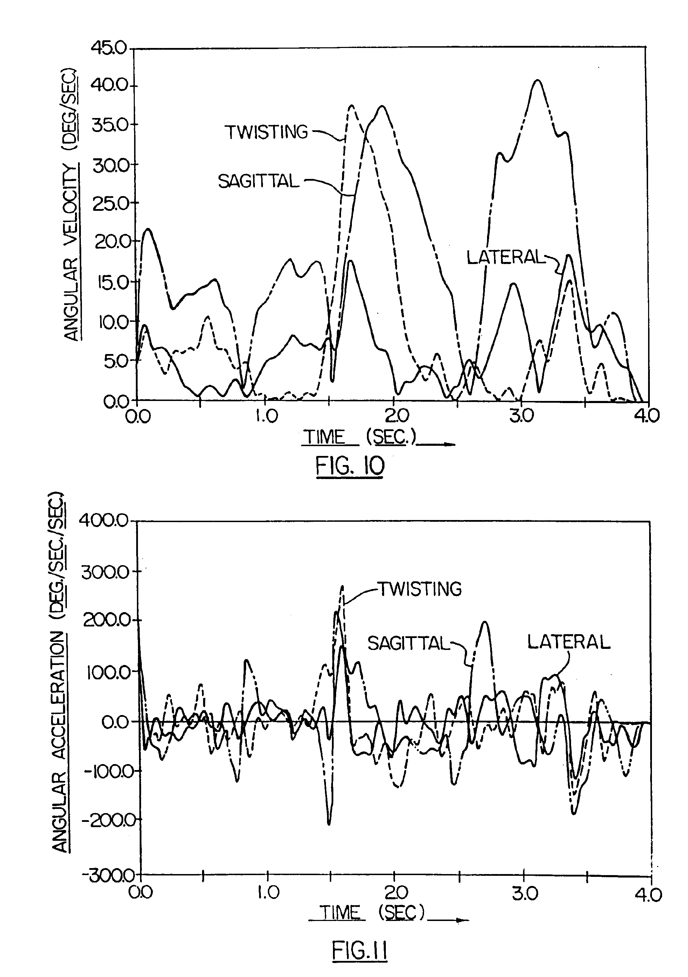

As schematically illustrated in Figure 8, the apparatus of the present invention may further include a multiplexing circuit 61 and a telemetry circuit 62 mounted on the base plate 40 of the apparatus. The multiplexing circuit 61 provides power to the four potentiometers, and sequentially mixes the signals from each of the potentiometers. The telemetry circuit 62 then transmits the mixed signals to a data acquisition computer 64 which demultiplexes the signal received from the telemetry circuit. Further, the data acquisition computer 64 may be programmed to smooth the data and convert the signals to angular position via a calibration, and then store the resulting data. The software then sorts the data for each of three planes of movement, namely the sagittal, transverse, and lateral planes. The motion characteristics in each plane include the angular position of the spine relative to the position to the pelvis considered as a function of time, and as illustrated in Figure 9. This information may then be differentiated to determine the angular velocity of the spine as a function of time and as illustrated in Figure 10, as well as the angular acceleration of the spine as a function of time and as illustrated in Figure 11.

As an alternative to the above described signal processing system, the potentiometers may be hardwired directly to the computer 64 as schematically illustrated by the dashed line 65 in Figure 8, thus bypassing the multiplexing circuit 61 and the telemetry circuit 62.

A calibration table is used to calibrate the apparatus of the present invention. Specifically, the apparatus is placed in the calibration table and the position of the apparatus is moved to a large number of different positions in space. The four potentiometer voltages associated with each of these positions is then recorded in the computer, and these voltages are then used to uniquely described the positions of the apparatus in space. A mathematical regression model is then used to estimate a best fit plane between the potentiometer voltages and the position in space of the apparatus. Therefore, when the apparatus is in use the voltage readings from the four potentiometers are compared to the model and the exact position of the apparatus in space is determined. As noted above, this information is then differentiated to determine instantaneous angular velocity and acceleration.

## Claims

1. An apparatus adapted to be mounted on the back of a patient and for monitoring the motion components of the spine during movement of the patient, and comprising an elongate exoskeleton (12) comprising plurality of elements (14) disposed in a longitudinally spaced apart arrangement, a bore (17) extending through each of said elements, with said bore of each element being aligned with the bores of the other elements to define a central longitudinal axis, a tubular spacer (22) disposed between each adjacent pair of elements for maintaining the separation thereof and with said spacers being coaxially aligned with said central longitudinal axis, at least two openings (18, 19, 20) extending longitudinally through each of said elements, and such that said openings of each element are longitudinally aligned with the corresponding openings of the remaining elements, support means for supporting said exoskeleton on the back of a patient and so that the exoskeleton is generally aligned along the spine, a cable (54) disposed coaxially along said central axis and through each of said central bores of said elements, and a connecting member (44) extending longitudinally through each of the aligned openings of said elements, with each of said connecting members being resiliently extensible in length, movement signaling means (50, 51, 52) mounted to said support means for detecting longitudinal movement of each of said connecting members caused by changes in length thereof, and for providing an output signal representative of such movement, and rotation signaling means (60) mounted to said support means for detecting rotation of said cable about said central axis in either direction and providing an output signal representative of such rotational movement,
characterised in that said support means comprises a top plate (30), strap means (29) for releasably securing said top plate on the upper back of the patient, a base plate (40), belt means (41) for releasably securing said base plate on the lower back of the patient, with one end of said cable being fixedly mounted to one of said top plate and base plate and rotatably mounted to the other of said top plate and base plate, and with the opposite ends of each connecting member being respectively attached to said top plate and said base plate, and wherein said support means further comprises a support plate (28) fixedly mounted to said strap means, and means (33) for slideably mounting said top plate to said support plate so as to permit limited relative movement in the longitudinal direction to thereby accommodate extension or contraction of the spine during use, and spring biasing means (35) for resiliently urging said top plate in a direction away from said base plate, and wherein said exoskeleton is not directly attached to the body of the patient along its length and flexing movement of the exoskeleton caused by the movement of the patient causes each of connecting members to selectively change in length.

2. The apparatus as defined in Claim 1 wherein said openings include first and second openings (18, 19) positioned on respective opposite lateral sides of said central axis, and a third opening (20) disposed above said central axis and equally spaced from said first and second openings, and such that said first, second, and third openings of each of said elements are disposed in a triangular arrangement, and whereby flexing of the exoskeleton in the lateral plane causes one or both the connecting members extending through said first and second openings to longitudinally change in length, and flexing of the exoskeleton in the sagittal plane causes the connecting members extending through said third openings to change in length.

3. The apparatus as defined in Claim 2 wherein each of said elements (14) is of generally T-shaped configuration having a horizontal base leg (15) and an upright leg (16), and wherein said central bore (17) is adjacent the intersection of said legs the first and second openings positioned at the respective ends of said horizontal leg, and with said third opening being positioned adjacent the free end of said upright leg.

4. The apparatus as defined in Claim 2 wherein said movement signaling means comprises three sensors (50, 51, 52) mounted to said other of said top plate and said base plate, with each of said sensors being operatively connected to one of said connecting members (44), and wherein said rotation signaling means comprises a fourth sensor (60) mounted to said other plate and being operatively connected to said cable.

5. The apparatus as defined in Claim 4 further comprising a transmitter (62) mounted to said support means and operatively connected to each of said sensors for transmitting the output signals thereof by telemetry, and a central computer (64) for receiving the transmitted signals from said transmitter and reproducing the same in visible form.

6. The apparatus as defined in Claim 1 wherein each of said connecting members comprises a non-extensible wire (45), and an extensible member (46) attached to one end of said wire.

7. The apparatus as defined in Claim 1 further comprising means (36, 55) mounting one end of said cable to said top plate so as to permit limited relative longitudinal movement between said one end and said top plate.

## Patentansprüche

1. Am Rücken eines Patienten anbringbare Vorrichtung zum Überwachen der Bewegungskomponenten der Wirbelsäule während Bewegungen des Patienten, mit einem langgestreckten Außenskelett (12), welches eine Vielzahl von in Längsrichtung im Abstand voneinander angeordneten Gliedern (14) aufweist, durch jedes von denen sich eine Bohrung (17) erstreckt, wobei die Bohrung jedes Gliedes mit den Bohrungen der anderen Glieder zur Bestimmung einer Längsmittelachse fluchtet, ein rohrförmiges Abstandselement (22), welches zwischen jeweils einem Paar benachbarter Glieder angeordnet ist, um sie voneinander getrennt zu halten, wobei die Abstandselemente mit der Längsmittelachse koaxial ausgerichtet sind, mindestens zwei Öffnungen (18, 19, 20), die sich in Längsrichtung derartig durch jedes der Glieder erstrecken, daß die Öffnungen jedes Gliedes mit den entsprechenden Öffnungen der übrigen Glieder in Längsrichtung fluchten, eine Stützeinrichtung zum Abstützen des Außenskeletss am Rücken eines Patienten auf solche Weise, daß das Außenskelett insgesamt längs der Wirbelsäule ausgerichtet ist, ein koaxial längs der Mittelachse und durch jede der zentralen Bohrungen in den Gliedern angeordnetes Kabel (54), sowie ein sich in Längsrichtung durch jede der miteinander fluchtenden Öffnungen der Glieder erstreckendes Verbindungselement (44), wobei jedes der Verbindungselemente federnd nachgiebig längsdehnbar ist, an der Stützeinrichtung angebrachte Bewegungssignalisiereinrichtungen (50, 51, 52) zum Erfassen einer durch Längenänderungen der Verbindungselemente hervorgerufenen Längsbewegung jedes der Verbindungselemente und zur Abgabe eines Ausgangssignals, welches eine derartige Bewegung wiedergibt, und eine an der Stützeinrichtung angebrachte Drehsignalisiereinrichtung (60) zum Erfassen einer Drehung des Kabels um die Mittelachse in der einen oder anderen Richtung sowie zur Abgabe eines Ausgangssignals, welches eine derartige Drehbewegung wiedergibt, **dadurch gekennzeichnet,** daß die Stützeinrichtung eine obere Platte (30), Gurtmittel (29) zum lösbaren Anbringen der oberen Platte am oberen Rücken des Patienten, eine untere Platte (40), Gürtelmittel (41) zum lösbaren Anbringen der unteren Platte am unteren Rücken des Patienten aufweist, wobei ein Ende des Kabels an einer der beiden Platten fest und an der anderen der beiden Platten drehbar angebracht ist und die entgegengesetzten Enden jedes Verbindungselements an der oberen bzw. unteren Platte festgemacht sind, und wobei die Stützeinrichtung ferner eine an den Gurtmitteln fest angebrachte Stützplatte (28) sowie Mittel (33) zum verschiebbaren Anbringen der oberen Platte an der Stützplatte, um eine begrenzte relative Bewegung in Längsrichtung zur Aufnahme von Dehnung und Kontraktion der Wirbelsäule im Gebrauch zu ermöglichen, und Federvorspannmittel (35) aufweist, die die obere Platte federnd nachgiebig in Richtung von der unteren Platte weg drängen, und wobei das Außenskelett nicht unmittelbar am Körper des Patienten längs seiner Länge befestigt ist und eine durch die Bewegung des Patienten verursachte Biegebewegung des Außenskeletts jedes der Verbindungselemente zu einer wahlweisen Längenänderung veranlaßt.

2. Vorrichtung nach Anspruch 1, bei der zu den Öffnungen erste und zweite Öffnungen (18, 19), die an entsprechenden, gegenüberliegenden, seitlichen Seiten der Mittelachse liegen, sowie eine dritte, oberhalb der Mittelachse und in gleichem Abstand von den ersten und zweiten Öffnungen vorgesehene Öffnung (20) gehören, die so angeordnet sind, daß die ersten, zweiten und dritten Öffnungen jedes der Glieder sich in einer Dreiecksanordnung befinden, wodurch beim Biegen des Außenskeletts in der Lateralebene eines oder beide der sich durch die ersten und zweiten Öffnungen erstreckenden Verbindungselemente zu einer Längenänderung in Längsrichtung veranlaßt werden und beim Biegen des Außenskeletts in der Sagittalebene die sich durch die dritten Öffnungen erstreckenden Verbindungselemente zu einer Längenänderung veranlaßt werden.

3. Vorrichtung nach Anspruch 2, bei der jedes der Glieder (14) insgesamt T-förmige Gestalt hat, mit einem horizontalen Basisschenkel (15) und einem aufrechten Schenkel (16), und bei der die mittlere Bohrung (17) sich in der Nähe der Kreuzung der Schenkel befindet, die ersten und zweiten Öffnungen an den jeweiligen Enden des horizontalen Schenkels und die dritte Öffnung sich in der Nähe des freien Endes des aufrechten Schenkels befindet.

4. Vorrichtung nach Anspruch 2, bei der die Bewegungssignalisiereinrichtung drei Sensoren (50, 51, 52) aufweist, die an der anderen der beiden, der oberen und unteren, Platten angebracht sind, wobei jeder der Sensoren betriebsmäßig mit einem der Verbindungselemente (44) verbunden ist, und bei der die Drehsignalisiereinrichtung einen vierten Sensor (60) aufweist, der an der anderen Platte angebracht und betriebsmäßig mit dem Kabel verbunden ist.

5. Vorrichtung nach Anspruch 4, ferner mit einem an der Stützeinrichtung angebrachten Sender (62), der mit jedem der Sensoren betriebsmäßig derartig verbunden ist, daß er deren Ausgangssignale durch Telemetrie überträgt, und mit einem Zentralrechner (64) zum Empfang der übertragenen Signale vom Sender und zur Wiedergabe derselben in sichtbarer Form.

6. Vorrichtung nach Anspruch 1, bei der jedes der Verbindungselemente einen nicht dehnbaren Draht (45) und ein an einem Ende des Drahtes befestigtes, dehnbares Element (46) aufweist.

7. Vorrichtung nach Anspruch 1, ferner mit Mitteln (36, 55), die ein Ende des Kabels so an der oberen Platte festlegen, daß eine begrenzte relative Längsbewegung zwischen dem einen Ende und der oberen Platte möglich ist.

## Revendications

1. Appareil destiné à être monté sur le dos d'un patient et à contrôler les composantes du mouvement de la colonne vertébrale pendant les mouvements du patient, et comprenant un exosquelette allongé (12) comportant plusieurs éléments (14) formant un ensemble espacé longitudinalement, un trou (17) placé dans chacun des éléments, le trou de chaque élément étant aligné sur les trous des autres éléments afin qu'un axe longitudinal central soit délimité, une entretoise tubulaire (22) disposée entre les éléments adjacents de chaque paire afin qu'ils restent séparés, les entretoises étant alignées coaxialement sur l'axe longitudinal central, au moins deux ouvertures (18, 19, 20) disposées longitudinalement dans chacun des éléments et de manière que les ouvertures de chaque élément soient alignées longitudinalement sur les ouvertures correspondantes des autres éléments, un dispositif de support de l'exosquelette sur le dos d'un patient de manière que l'exosquelette soit aligné de façon générale le long de la colonne vertébrale, un câble (54) disposé coaxialement le long de l'axe central et dans tous les trous centraux des éléments, et un organe (44) de connexion disposé longitudinalement dans toutes les ouvertures alignées des éléments, chacun des organes de connexion étant extensible élastiquement dans la direction de la longueur, un dispositif (50, 51, 52) de signalisation de mouvement, monté sur le dispositif de support et destiné à détecter un mouvement longitudinal de chacun des organes de connexion provoqué par ses changements de longueur et à transmettre un signal de sortie représentatif de ce mouvement, et un dispositif (60) de signalisation de rotation monté sur le dispositif de support et destiné à détecter la rotation du câble autour de l'axe central dans n'importe quel sens et à donner un signal de sortie représentatif du mouvement en rotation,
caractérisé en ce que le dispositif de support comporte une plaque supérieure (30), un dispositif (29) à sangle de fixation temporaire de la plaque supérieure sur la partie supérieure du dos du patient, une plaque de base (40), un dispositif à ceinture (41) destiné à fixer temporairement la plaque de base sur la partie inférieure du dos du patient, une première extrémité du câble étant montée par fixation sur l'une des plaques supérieure et de base de manière qu'il puisse tourner sur l'autre des plaques supérieure et de base, les extrémités opposées de chaque organe de connexion étant fixées respectivement à la plaque supérieure et à la plaque de base, et le dispositif de support comporte en outre une plaque de support (28) montée à demeure sur le dispositif à sangle, et un dispositif (33) de montage coulissant de la plaque supérieure sur la plaque de support de manière qu'il permette un mouvement relatif limité en direction longitudinale et compense ainsi l'allongement ou la contraction de la colonne vertébrale pendant l'utilisation, et un dispositif (35) de rappel à ressort destiné à repousser élastiquement la plaque supérieure du côté opposé à la plaque de base, et dans lequel l'exosquelette n'est pas directement fixé au corps du patient sur sa longueur et le mouvement de flexion de l'exosquelette provoqué par le mouvement du patient provoque un changement sélectif de longueur de chacun des organes de connexion.

2. Appareil selon la revendication 1, dans lequel les ouvertures comprennent des première et seconde ouvertures (18, 19) disposées sur les côtés latéraux opposés respectifs de l'axe central, et une troisième ouverture (20) placée au-dessus de l'axe central et également espacée de la première et de la seconde ouverture, de manière que la première, la seconde et la troisième ouverture de chacun des éléments forment un ensemble triangulaire et de manière que la flexion de l'exosquelette dans le plan latéral provoque l'entrée de l'un au moins des organes de connexion dans la première et la seconde ouverture pour changer longitudinalement de longueur, et la flexion de l'exosquelette dans le plan sagittal provoque la pénétration des organes de connexion dans les troisièmes ouvertures afin qu'il puisse changer de longueur.

3. Appareil selon la revendication 2, dans lequel chacun des éléments (14) a une configuration générale en T ayant une branche horizontale de base (15) et une branche verticale (16), et dans lequel dans le trou central (17) est adjacent à l'intersection des branches, la première et la seconde ouverture étant placées aux extrémités respectives de la branche horizontale, la troisième ouverture étant adjacente à l'extrémité libre de la branche verticale.

4. Appareil selon la revendication 2, dans lequel le dispositif de signalisation de mouvement comprend trois capteurs (50, 51, 52) montés sur l'autre des plaques supérieure et de base, chacun des capteurs étant connecté lors du fonctionnement à l'un des organes de connexion (44), et le dispositif de signalisation de rotation comporte un quatrième capteur (60) monté sur l'autre plaque et connecté au câble pendant le fonctionnement.

5. Appareil selon la revendication 4, comprenant en outre un émetteur (62) monté sur le dispositif de support et connecté à chacun des capteurs pendant le fonctionnement afin qu'il transmette leurs signaux de sortie par télémétrie, et un ordinateur central (64) destiné à recevoir les signaux émis par l'émetteur et à les reproduire sous forme visible.

6. Appareil selon la revendication 1, dans lequel chacun des organes de connexion comprend un fil inextensible (45), et un organe extensible (46) fixé à une extrémité du fil.

7. Appareil selon la revendication 1, comprenant en outre un dispositif (36, 55) de montage d'une première extrémité du câble sur la plaque supérieure afin qu'il permette un mouvement longitudinal relatif limité entre la première extrémité et la plaque supérieure.
